# EUROPÄISCHE PATENTSCHRIFT

(11) **EP 2 117 628 B1**
(45) Veröffentlichungstag und Bekanntmachung des Hinweises auf die Patenterteilung: **06.11.2013**
(21) Anmeldenummer: 08716528.8
(22) Anmeldetag: 13.03.2008
(51) Int. Cl.: A61M 16/04

(54) **TRACHEAL- ODER TRACHEOSTOMIETUBENANORDNUNG**
TRACHEAL OR TRACHEOSTOMY TUBULAR ARRANGEMENT
SYSTÈME DE TUBES TRACHÉAL OU DE TRACHÉOSTOMIE

(30) Priorität: 13.03.2007 DE 102007011930
(43) Veröffentlichungstag der Anmeldung: 18.11.2009
(73) Patentinhaber: Willy Rüsch GmbH, 71394 Kernen-Rommelshausen (DE)
(72) Erfinder: SINGVOGEL, Armin, 71686 Remsbeck (DE)
(74) Vertreter: Grünecker, Kinkeldey, Stockmair & Schwanhäusser
(86) Internationale Anmeldenummer: PCT/EP2008/002032
(87) Internationale Veröffentlichungsnummer: WO 2008/110377

(56) Entgegenhaltungen:
- EP-A- 0 920 881
- WO-A-91/12844
- WO-A-2004/101048
- DE-C1- 19 707 364
- US-A- 2 923 299
- US-A- 3 948 274
- US-A- 5 546 936

## Beschreibung

Die Erfindung betrifft eine Tracheal- oder Tracheostomietubenanordnung mit einem äußeren Tubus und einer inneren Kanüle, welche in den äußeren Tubus einschiebbar und aus diesem herausziehbar ist, wobei die innere Kanüle mindestens eine zumindest ihre innere Oberfläche ausbildende Kunststoffschicht und eine die Kunststoffschicht stützende Stützstruktur aufweist.

Tracheal- oder Tracheostomietubenanordnungen werden unter anderem bei der künstlichen Beatmung von Patienten und in der Intensivmedizin eingesetzt. Eine Tracheostomietubenanordnung wird dabei durch einen Luftröhrenschnitt in die Luftröhre eingesetzt, während eine Trachealtubenanordnung durch den Mund oder durch die Nase des Patienten in die Luftröhre eingesetzt wird. Tracheal- und Tracheostomietubenanordnungen unterscheiden sich im Wesentlichen lediglich durch ihre an ein Beatmungsgerät anschließbare maschinenseitige Endbereiche, da diese Endbereiche an unterschiedlichen Körperteilen zu fixieren sind.

Gattungsgemäße Tracheal- oder Tracheostomietubenanordnungen sind zum Beispiel aus der WO 91/12844 A1 bekannt. Der äußere Tubus (Außentubus) einer derartigen Tracheal- oder Tracheostomietubenanordnung ist zum Einsetzen in die Luftröhre vorgesehen. Die innere Kanüle wird durch den äußeren Tubus eingeschoben und kann in periodischen Abständen entfernt und ersetzt werden, nämlich dann, wenn sich in der Kanüle so viel Sekret angesammelt hat, dass das Ersetzen als zweckdienlich erachtet wird.

Das Einschieben der inneren Kanüle in den Tubus kann jedoch schwierig sein. Um einen möglichst großen Innendurchmesser der inneren Kanüle und damit einen möglichst geringen Atmungswiderstand zu erreichen, sollte die innere Kanüle möglichst dünnwandig sein und der Außendurchmesser der inneren Kanüle sollte im Wesentlichen dem Innendurchmesser des Tubus entsprechen. Wegen des Durchmesserverhältnisses der Innenkanüle zum Tubus liegt die Innenkanüle beim Einschieben zumeist großflächig an der Innenwand des Tubus an. Daraus folgt ein großer Reibungswiderstand beim Einschieben.

Wegen dieses Reibungswiderstandes und der Dünnwandigkeit sowie der Flexibilität der Innenkanüle kommt es beim Einschieben häufig zu einem Abknicken der Innenkanüle. Zur Lösung dieses Problems schlägt die WO 91/12844 A1 vor, um die Außenoberfläche der inneren Kanüle herum ein Filament z.B. aus Metal spiralförmig anzuordnen. Mit einer derartigen Außenspirale wird die radiale Steifigkeit der inneren Kanüle erhöht und der Reibungskoeffizient zwischen Innenwand des Tubus und Außenoberfläche der inneren Kanüle vermindert.

Um eine ausreichende Steifigkeit zu erreichen muss jedoch die Materialstärke der Außenspirale relativ groß sein, so dass der Innendurchmesser der inneren Kanüle unter Erhöhung des Atemwiderstandes entsprechend abnimmt.

Der Erfindung liegt die Aufgabe zugrunde, eine Tracheal- oder Tracheostomietubenanordnung und ein Herstellungsverfahren der Innenkanüle der Tracheal- oder Tracheostomietubenanordnung bereitzustellen, welche die Nachteile des Standes der Technik vermeiden, wobei insbesondere die Knickstabilität bei dünnwandigen Innenkanülen verbessert werden soll.

Diese Aufgabe wird durch die Tracheal- oder Tracheostomietubenanordnung des unabhängigen Anspruchs gelöst. Die abhängigen Ansprüche stellen bevorzugte Ausführungsformen der Erfindung dar.

Eine erfindungsgemäße Tracheal- oder Tracheostomietubenanordnung umfasst einen äußeren Tubus und eine inneren Kanüle, welche in den äußeren Tubus einschiebbar und aus diesem herausziehbar ist. Dabei weist die innere Kanüle mindestens eine zumindest ihre innere Oberfläche ausbildende Kunststoffschicht und eine die Kunststoffschicht stützende Stützstruktur auf. Erfindungsgemäß ist die Stützstruktur von einem schlauchförmigen Geflecht aus Fäden und/oder Fasern ausgebildet.

Die Kunststoffschicht bildet zusammen mit dem schlauchförmigen Geflecht einen geschlossenen Schlauch aus. Dadurch, dass die innere Oberfläche von der Kunststoffschicht ausgebildet wird, ist diese relativ glatt, so dass Atemsekrete anhaften können und nicht durch sich an Rauhigkeiten ausbildende Turbulenzen der Atemluftströmung abgelöst werden. Da ein Geflecht aus vielen miteinander verwobenen Fäden besteht, können die Fäden sehr viel dünner als bei einer lediglich spiralförmigen Stützstruktur ausgebildet sein. Die Innenkanüle kann damit sehr dünnwandig ausgebildet sein. Weiter hat ein Geflecht den Vorteil, dass eine Stabilisierung in mehreren Richtungen erfolgt. Das Geflecht kann z.B. aus einer Vielzahl von ineinander verwobenen, sich kreuzenden spiralförmig angeordneten Fäden ausgebildet sein. Derart sind zwischen benachbarten Fäden von den Fäden gegeneinander abgegrenzte rautenförmige Öffnungen in der Stützstruktur vorhanden. Die Kunststoffschicht muss also jeweils nur diese rautenförmigen Öffnungen überspannen, wogegen bei einer Stützstruktur aus lediglich einem spiralförmig angeordneten Filament ein zusammenhängender Bereich über die gesamte Länge der Innenkanüle überspannt werden muss. Die Kunststoffschicht kann also bei gleicher Steifigkeit wesentlich dünner ausgebildet sein. Die erfindungsgemäß ausgebildete Innenkanüle ist daher bei einer sehr geringen Wandstärke knickstabil und flexibel.

Bevorzugt ist die Stützstruktur von der Kunststoffschicht im Wesentlichen vollständig umhüllt, derart, dass auch die äußere Oberfläche der inneren Kanüle von der Kunststoffschicht ausgebildet ist. Eine derartige Innenkanüle kann einfach z.B. durch bei Schläuchen übliches Einarbeiten einer Stützstruktur in Kunststoff hergestellt werden.

Besonders bevorzugt ist die von der Kunststoffschicht ausgebildete innere Oberfläche der inneren Kanüle im Wesentlichen glatt und die von der Kunststoffschicht ausgebildete äußere Oberfläche bildet die Außenstruktur des Geflechts ab. Die abgebildete Außenstruktur des Geflechts bildet eine relativ zur inneren Oberfläche der inneren Kanüle raue Oberfläche aus. Eine derartige raue Oberfläche hat beim Einschieben der Innenkanüle in den Tubus weniger Kontakt zur Innenoberfläche des Tubus, so dass die Reibung zwischen diesen Oberflächen geringer ist als bei glatten Oberflächen.

Wenn die Fäden oder Fasern des Geflechts aus Kunststoff und nicht z.B. aus Metall, was ebenfalls möglich wäre, bestehen, ist ein besonders geringes Gewicht bei großer Stabilität der inneren Kanüle erreichbar. Die das Geflecht bildenden Fäden oder Fasern können z.B. aus Polyester, Trevira, Polyamid, Polyethylen, Polyimid, Polyethersulfon, Copolyamid oder Fluorcarbon bestehen. Es können auch Fäden oder Fasern unterschiedlicher Materialien miteinander verflochten sein. Bevorzugt wird jedoch ein Polyestergeflecht verwendet. Die Fadenstärke der Kunststofffäden kann z.B. 0,05 bis 0,3 mm betragen. Weiter ist bei einem Kunststoffgeflecht ein leichtes Einbringen von Zusatzfunktionen wie Schlauchöffnungen zur Sprachermöglichung bei bestehender Tubuseinlage, also, wenn der Tubus in der Luftröhre eines Patienten appliziert ist, möglich.

Die Fäden oder Fasern des Geflechts sind vorteilhaft zumindest im Bereich der freien Enden der inneren Kanüle offen oder Verbunden und werden als ganzes Teil mit Wärme behandelt um ein Aufspringen der Enden zu verhindern.

Gleichfalls ist ein Verschweißen, laminieren oder Verkleben möglich.

Die Enden können auch durch einen dünnen Film geschützt werden der durch einen so genannten dünnen flexiblen Schrumpfschlauch erzeugt wird.

Eine weitere Möglichkeit die Endfäden von dem Aufspringen zu schützen ist ein formschlüssiges dünnes Teil ähnlich einem Clipverschluss mit einem Innen- und Außenring zu verwenden. Diese Möglichkeit kann auch auf ausgestanzte Augen und/oder Löcher angewandt werden.

Vorteilhaft besteht die Kunststoffschicht z.B. aus Silikonkautschuk oder einem anderen Elastomer wie TPE, PUR oder PVC. Eine derartige Kunststoffschicht lässt sich einfach durch Tauchen eines schlauchförmigen Geflechts als Stützstruktur in eine flüssige Silikonkautschukmasse auf die Stützstruktur aufbringen.

Weiter können an einer derartigen inneren Kanüle Hilfsmittel aus Silikonkautschuk im Materialverbund vorgesehen sein. Es kann z.B. ein Griff am maschinenseitigen Endbereich der inneren Kanüle angeformt sein.

Bevorzugt ist als Hilfsmittel an einem der freien Enden der inneren Kanüle ein Anschlag vorgesehen, wobei der Anschlag z.B. von einem auf die Außenoberfläche der inneren Kanüle mit seiner Innenoberfläche mittels Silikonkleber aufgeklebten Silikonschlauchstück ausgebildet sein kann. Ein derartiger Anschlag ermöglicht ein passgenaues Positionieren der Inneren Kanüle im Außentubus. Dabei ist der Anschlag so gestaltet, dass eine weitere Adaption an den äußeren Konnektor nicht gestört wird.

Vorteilhaft kann in der Kunststoffschicht und/oder in dem Geflecht ein Röntgenschatten gebender Füllstoff vorgesehen sein, und/oder es kann zumindest die innere Oberfläche der inneren Kanüle eine antimikrobiel wirksame Beschichtung, insbesondere eine Silberbeschichtung, aufweisen.

Bei einem erfindungsgemäßen Verfahren zur Herstellung einer inneren Kanüle für eine Tracheal- oder Tracheostomietubenanordnung, bei der die Stützstruktur der inneren Kanüle von der Kunststoffschicht im Wesentlichen vollständig umhüllt ist (geschlossene Innenkanülenbeschichtung), derart, dass auch die äußere Oberfläche der inneren Kanüle von der Kunststoffschicht ausgebildet ist, wird eine schlauchförmige Stützstruktur von einem Geflecht aus Fäden und/oder Fasern bereitgestellt, und die Kunststoffschicht durch Tauchen des Geflechts in einen Flüssigkunststoff, bevorzugt Flüssigsilikonkautschuk, und Aushärten der Kunststoffschicht hergestellt. Die geschlossene Innenkanülenbeschichtung erfolgt bevorzugt durch lediglich einmaliges Eintauchen, was zu einer besonders dünnen geschlossenen Beschichtung führt. Dabei wird kein Kernstab verwendet. Einfaches Tauchen ist wesentlich schneller und unkomplizierter als Extrusionsverfahren zur Kunststoffbeschichtung eines Geflechts. Das erfindungsgemäße Verfahren ist somit sehr kostengünstig.

Ähnliche Schläuche können auch im Spritzgußverfahren durch Einlegen des Geflechts oder durch Besprühen des Geflechts hergestellt werden.

Durch Tauchen kann bei geeigneter Wahl der Geometrie des Geflechts der Stützstruktur ohne weitere Maßnahmen erreicht werden, dass die von der Kunststoffschicht ausgebildete innere Oberfläche der inneren Kanüle im Wesentlichen glatt ist und die von der Kunststoffschicht ausgebildete äußere Oberfläche die Außenstruktur des Geflechts abbildet. Derart ist eine glatte Innenbeschichtung bei rauer Außenbeschichtung direkt erreichbar, die das Ein- und Ausführen der inneren Kanüle in den Tubus sehr erleichtert, so dass keine Blockung durch Abknicken der Innenkanüle auftritt. Die genannte Oberflächenstruktur kann dadurch entstehen, dass die innere Oberfläche einen geringeren Radius aufweist als die äußere Oberfläche. Durch die Oberflächenspannung des Flüssigkunststoffes wird die noch flüssige Kunststoffschicht im Bereich der Zwischenräume zwischen benachbarten Fäden bzw. Fasern des Geflechts radial nach innen gezogen. Die von der Kunststoffschicht zwischen benachbarten Fasern ausgebildete Membran verläuft daher im Bereich des inneren Radius der inneren Kanüle, was zu der genannten Oberflächenstruktur führt. Wenn das Geflecht aus sich überkreuzenden parallel verlaufenden Fäden besteht, so dass zwischen benachbarten Fäden rautenförmige Zwischenräume bestehen, hat sich bei Verwendung von Silikonkautschuk dazu ein Fadenabstand von 20 Geflechtsrauten im Bereich von 13 plus/minus 5 mm als zweckmäßig erwiesen. Die Geflechtrauten haben also einen Durchmesser im Bereich von ca. 1 mm.

Es versteht sich von selbst, dass auch zur Herstellung einer inneren Kanüle Fäden und Geflechte verwendet werden können, deren Querschnitte so ausgeformt sind, dass sie zur Innenoberfläche ausgerichtet abgeflacht, eben und zur Außenoberfläche ausgerichtet gebogen, gekrümmt ausgebildet sind.

Die Erfindung wird nachfolgend anhand eines Ausführungsbeispiels unter Bezugnahme auf die Zeichnungen näher erläutert.
Figur 1 zeigt eine erfindungsgemäße Tracheostomietubenanordnung;
Figur 2 zeigt die innere Kanüle der erfindungsgemäßen Tracheostomietubenanordnung aus Figur 1;
Figur 3 zeigt eine vergrößerte Querschnittsdarstellung eines Teils der inneren Kanüle der erfindungsgemäßen Tracheostomietubenanordnung aus Figur 1;

Die Figuren der Zeichnungen zeigen den erfindungsgemäßen Gegenstand stark schematisiert und sind nicht maßstäblich zu verstehen. Die einzelnen Bestandteile des erfindungsgemäßen Gegenstandes sind so dargestellt, dass ihr Aufbau gut gezeigt werden kann.

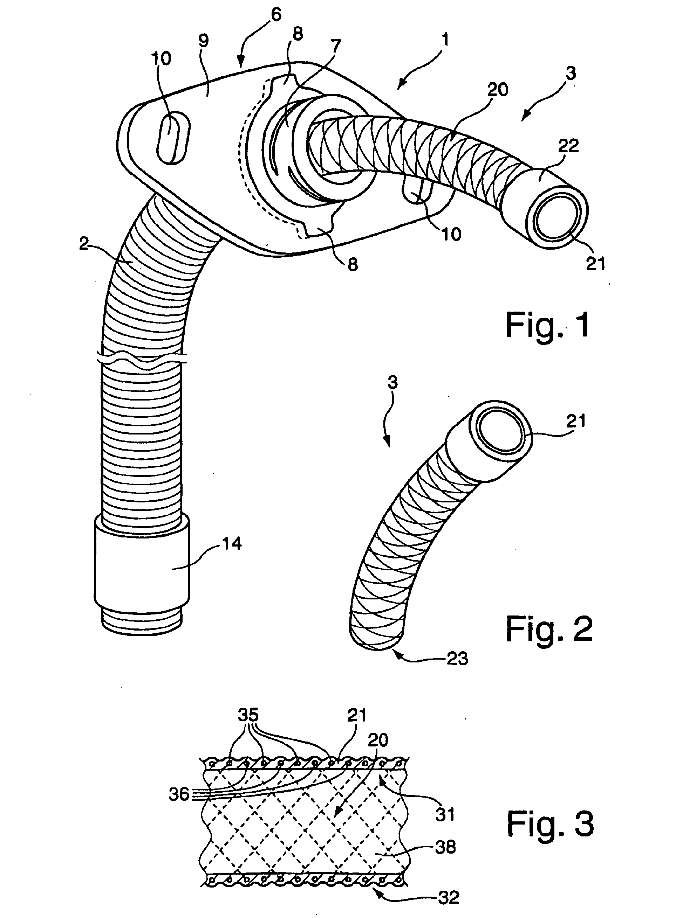

In Figur 1 ist eine erfindungsgemäße Tracheostomietubenanordnung 1 gezeigt. Die Tracheostomietubenanordnung 1 umfasst einen äußeren Tubus 2 und eine innere Kanüle 3. Die innere Kanüle 3 ist in den äußeren Tubus 2 einschiebbar und aus diesem herausziehbar. In der Figur ist die innere Kanüle 3 teilweise in den Tubus 2 eingeschoben. Der Tubus 2 weist an seinem maschinenseitigen Ende, welches an ein Atemgerät anschließbar ist, einen Flansch 6 auf. Am Flansch 6 sind ein Schraubgewinde 7 und Arretierungen 8 ausgebildet, wodurch ein verrastendes Einschrauben eines Anschlusselements eines Beatmungsrohrsystems des Atemgeräts möglich ist. Weiter ist am Flansch 6 eine Manschette 9 mit Öffnungen 10 zur Befestigung der Manschette 9 am Hals eines Patienten vorgesehen. Im Bereich des in der Luftröhre des Patienten anzuordnenden patientenseitigen Endes des Tubus 2 ist weiter eine aufblasbare Manschette 14 angeordnet. Diese Manschette 14 kann ballonartig von außen aufgeblasen werden, um den Tubus 2 ortsfest in der Luftröhre zu fixieren. Der Tubus 2 selbst ist z.B. von einem Kunststoffschlauch ausgebildet, wobei in den Kunststoff eine z.B. Metallspirale eingearbeitet ist.

Die innere Kanüle 3 weist eine von einem schlauchförmigen Geflecht 20 aus Kunststofffäden ausgebildete Stützstruktur auf. Diese Stützstruktur ist in eine Kunststoffschicht 21 eingegossen, die die innere und die äußere Oberfläche der inneren Kanüle 3 ausbildet. Die Stützstruktur ist also von der Kunststoffschicht 21 vollständig umhüllt, so dass ein dichter Schlauch ausgebildet ist. Als Hilfsmittel ist am maschinenseitigen freien Enden der inneren Kanüle ein Anschlag 22 vorgesehen. Dieser Anschlag 22 ist von einem auf die Außenoberfläche der inneren Kanüle mit seiner Innenoberfläche mittels Silikonkleber aufgeklebten kurzen Silikonschlauchstück ausgebildet.

In Figuren 2 ist die innere Kanüle 3 der erfindungsgemäßen Tracheostomietubenanordnung aus Figur 1 gezeigt. Das in Figur 1 durch den Tubus verdeckte patientenseitige Ende 23 der inneren Kanüle 3 weist keine besondere Ausformung gegenüber dem Schlauch auf, der durch die, mit der Kunststoffschicht 21 umhüllte, Stützstruktur ausgebildet ist. Dieses patientenseitige Ende kann einfach als Schnittkante dieses Schlauches ausgebildet sein.

In Figur 3 ist eine vergrößerte Querschnittsdarstellung eines Teils der inneren Kanüle der erfindungsgemäßen Tracheostomietubenanordnung aus Figur 1 gezeigt. Die von der Kunststoffschicht 21 ausgebildete innere Oberfläche 31 der inneren Kanüle ist glatt und die von der Kunststoffschicht 21 ausgebildete äußere Oberfläche 32 bildet die Außenstruktur des Geflechts 20 ab. Die äußere Oberfläche 32 ist also rauer als die innere Oberfläche 31. Das Geflecht 20 besteht aus sich überkreuzenden parallel verlaufenden Fäden 35,36, so dass zwischen benachbarten Fäden rautenförmige Zwischenräume 38 bestehen. D.h., ein Teil der Fäden 35 verläuft in einer links orientierten Spirale um die Längsachse der inneren Kanüle und ein anderer Teil der Fäden 36 verläuft in einer rechts orientierten Spirale um die Längsachse der inneren Kanüle. Die jeweils links oder rechts orientiert verlaufenden Fäden 35,36 sind jeweils parallel und die unterschiedlich orientiert verlaufenden Fäden kreuzen sich. Die Fäden 35,36 können dabei bevorzugt verwoben sein, so dass von einem Faden gekreuzte benachbarte Fäden abwechselnd von unten, d.h. innenwandseitig, und von oben, d.h. außenwandseitig, überkreuzt sind. Es ist jedoch auch möglich z.B. alle links orientiert verlaufenden Fäden innenwandseitig anzuordnen und alle rechts orientiert verlaufende Fäden außenwandseitig anzuordnen oder umgekehrt.

Vorgeschlagen wird eine Tracheal- oder Tracheostomietubenanordnung 1 mit einem äußeren Tubus 2 und einer inneren Kanüle 3, welche in den äußeren Tubus 2 einschiebbar und aus diesem herausziehbar ist, wobei die innere Kanüle 3 mindestens eine zumindest ihre innere Oberfläche 31 ausbildende Kunststoffschicht 21 und eine die Kunststoffschicht 21 stützende Stützstruktur aufweist. Dabei ist die Stützstruktur von einem schlauchförmigen Geflecht 20 aus Fäden 35,36 und/oder Fasern ausgebildet.

Die Erfindung beschränkt sich nicht auf die vorstehend angegebenen Ausführungsbeispiele. Vielmehr ist eine Anzahl von Varianten denkbar, welche auch bei grundsätzlich anders gearteter Ausführung von den Merkmalen der Erfindung Gebrauch machen.

## Patentansprüche

1. Tracheal- oder Tracheostomietubenanordnung (1) mit einem äußeren Tubus (2) und einer inneren Kanüle (3), welche in den äußeren Tubus (2) einschiebbar und aus diesem herausziehbar ist, wobei die innere Kanüle (3) mindestens eine zumindest ihre innere Oberfläche (31) ausbildende Kunststoffschicht (21) und eine die Kunststoffschicht (21) stützende Stützstruktur aufweist, und die Stützstruktur von einem schlauchförmigen Geflecht (20) aus Fäden (35,36) und/oder Fasern ausgebildet ist,
**dadurch gekennzeichnet, dass**
die Stützstruktur von der Kunststoffschicht (21) im Wesentlichen vollständig umhüllt ist, derart, dass auch die äußere Oberfläche (32) der inneren Kanüle (3) von der Kunststoffschicht (21) ausgebildet ist.

2. Tracheal- oder Tracheostomietubenanordnung nach Anspruch 1, **dadurch gekennzeichnet, dass** die von der Kunststoffschicht (21) ausgebildete innere Oberfläche (31) der inneren Kanüle (3) im Wesentlichen glatt ist und die von der Kunststoffschicht (21) ausgebildete äußere Oberfläche (32) die Außenstruktur des Geflechts (20) abbildet.

3. Tracheal- oder Tracheostomietubenanordnung nach Anspruch 1 oder 2, **dadurch gekennzeichnet, dass** die Fäden (35,36) oder Fasern des Geflechts (20) aus Kunststoff bestehen.

4. Tracheal- oder Tracheostomietubenanordnung nach Anspruch 3, **dadurch gekennzeichnet, dass** die Fäden (35,36) oder Fasern des Geflechts (20) zumindest im Bereich der freien Enden der inneren Kanüle offen, verschweißt, umhüllt und/oder laminiert sind.

5. Tracheal- oder Tracheostomietubenanordnung nach einem der Ansprüche 1 bis 4, **dadurch gekennzeichnet, dass** die Kunststoffschicht (21) aus Silikonkautschuk besteht.

6. Tracheal- oder Tracheostomietubenanordnung nach Anspruch 5, **dadurch gekennzeichnet, dass** an der inneren Kanüle Hilfsmittel aus Silikonkautschuk im Materialverbund vorgesehen sind.

7. Tracheal- oder Tracheostomietubenanordnung nach Anspruch 6, **dadurch gekennzeichnet, dass** als Hilfsmittel an einem der freien Enden der inneren Kanüle (3) ein Anschlag (22) vorgesehen ist, wobei der Anschlag (22) von einem auf die Außenoberfläche (32) der inneren Kanüle (3) mit seiner Innenoberfläche bevorzugt mittels Silikonkleber aufgeklebten Silikonschlauchstück ausgebildet ist, bevorzugt wobei der Anschlag derart ausgebildet ist, dass eine weitere Adaption von Anschlussteilen und/oder Hilfsmitteln durch den Anschlag nicht behindert wird.

8. Tracheal- oder Tracheostomietubenanordnung nach einem der Ansprüche 1 bis 7, **dadurch gekennzeichnet, dass** in der Kunststoffschicht (21) und/oder in dem Geflecht (20) ein Röntgenschatten gebender Füllstoff vorgesehen ist und/oder, dass zumindest die innere Oberfläche (31) der inneren Kanüle (3) eine antimikrobiel wirksame Beschichtung, insbesondere eine Silberbeschichtung aufweist.

9. Verfahren zur Herstellung einer inneren Kanüle für eine Tracheal- oder Tracheostomietubenanordnung nach mindestens einem der Ansprüche 1 oder 2,
**dadurch gekennzeichnet, dass**
eine schlauchförmige Stützstruktur aus einem Geflecht (20) aus Fäden (35,36) und/oder Fasern bereitgestellt wird, und
die Kunststoffschicht (21) durch Tauchen des Geflechts (20) in einen Flüssigkunststoff, bevorzugt Flüssigsilikonkautschuk, und Aushärten der Kunststoffschicht (21) hergestellt wird.

## Claims

1. Tracheal or tracheostomy tube arrangement (1), having an outer tube (2) and an inner cannula (3) which can be inserted in the outer tube (2) and can be withdrawn therefrom, the inner cannula (3) having at least one layer (21) of plastics or elastomeric material which forms at least its inner surface (31) and having a supporting structure supporting the layer (21) of plastics or elastomeric material, and the supporting structure being formed by a tubular braided structure (20) of filaments (35, 36) and/or fibres, **characterised in that** the supporting structure is substantially completely enclosed by the layer (21) of plastics or elastomeric material in such a way that the outer surface (32) too of the inner cannula (3) is formed by the layer (21) of plastics or elastomeric material.

2. Tracheal or tracheostomy tube arrangement according to claim 1, **characterised in that** the inner surface (31) of the inner cannula (3) which is formed by the layer (21) of plastics or elastomeric material is substantially smooth and the outer surface (32) which is formed by the layer (21) of plastics or elastomeric material reproduces the external structure of the braided structure (20).

3. Tracheal or tracheostomy tube arrangement according to claim 1 or 2, **characterised in that** the filaments (35, 36) or fibres of the braided structure (20) consist of plastics or elastomeric material.

4. Tracheal or tracheostomy tube arrangement according to claim 3, **characterised in that** the filaments (35, 36) or fibres of the braided structure (20) are exposed, welded, sheathed and/or laminated, at least in the region of the free ends of the inner cannula.

5. Tracheal or tracheostomy tube arrangement according to one of claims 1 to 4, **characterised in that** the layer (21) of plastics or elastomeric material consists of silicone rubber.

6. Tracheal or tracheostomy tube arrangement according to claim 5, **characterised in that** physically united ancillary items of silicon rubber are provided on the inner cannula.

7. Tracheal or tracheostomy tube arrangement according to claim 6, **characterised in that** a stop (22) is provided as an ancillary item on one of the free ends of the inner cannula (3), the stop (22) being formed by a piece of silicone tubing which is bonded by its inner surface to the outer surface (32) of the inner cannula (3) preferably by means of silicone adhesive, and the stop preferably being so formed that it does not hinder any further adaptation of connecting parts and/or ancillary items.

8. Tracheal or tracheostomy tube arrangement according to one of claims 1 to 7, **characterised in that** a filler which casts an X-ray shadow is provided in the layer (21) of plastics or elastomeric material and/or in the braided structure (20), and/or **in that** at least the inner surface (31) of the inner cannula (3) has a coating having an anti-microbial action and in particular a silver coating.

9. Method of producing an inner cannula for a tracheal or tracheostomy tube arrangement according to at least one of claims 1 and 2, **characterised in that** a tubular supporting structure comprising a braided structure (20) of filaments (35, 36) and/or fibres is provided, and the layer (21) of plastics or elastomeric material is produced by dipping the braided structure (20) in a liquid plastics or elastomeric material, and preferably in liquid silicone rubber, and curing the layer of plastics or elastomeric material.

## Revendications

1. Agencement de tube trachéal ou de trachéostomie (1) avec un tube externe (2) et une canule interne (3) qui peut être insérée dans le tube externe (2) et peut être extraite de celui-ci, dans lequel la canule interne (3) comporte au moins une couche en matériau synthétique (21) qui constitue au moins sa surface interne (31) et une structure de support qui porte la couche en matériau synthétique (21), et dans lequel la structure de support est constituée d'une tresse tubulaire (20) en filaments (35, 36) et/ou en fibres,
**caractérisé**
**en ce que** la structure de support est essentiellement entièrement enveloppée par la couche en matériau synthétique (21), de telle sorte que la surface externe (32) de la canule interne (3) est aussi constituée par la couche en matériau synthétique (21).

2. Agencement de tube trachéal ou de trachéostomie selon la revendication 1, **caractérisé en ce que** la surface interne (31) de la canule interne (3) constituée par la couche en matériau synthétique (21) est essentiellement lisse, et la surface externe (32) constituée par la couche en matériau synthétique (21) reproduit la structure externe de la tresse (20).

3. Agencement de tube trachéal ou de trachéostomie selon la revendication 1 ou 2, **caractérisé en ce que** les filaments (35, 36) ou les fibres de la tresse (20) sont constitués d'une matière synthétique.

4. Agencement de tube trachéal ou de trachéostomie selon la revendication 3, **caractérisé en ce que** les filaments (35, 36) ou les fibres de la tresse (20) sont ouverts, soudés, enveloppés et/ou laminés au moins dans la zone des extrémités libres de la canule interne.

5. Agencement de tube trachéal ou de trachéostomie selon l'une des revendications 1 à 4, **caractérisé en ce que** la couche en matériau synthétique (21) est constituée d'un caoutchouc de silicone.

6. Agencement de tube trachéal ou de trachéostomie selon la revendication 5, **caractérisé en ce que** des accessoires en caoutchouc de silicone liés au matériau sont pourvus sur la canule interne.

7. Agencement de tube trachéal ou de trachéostomie selon la revendication 6, **caractérisé en ce qu'**une butée (22) est pourvue comme accessoire à une des extrémités libres de la canule interne (3), moyennant quoi la butée (22) est constituée par un morceau de tube de silicone collé sur la surface externe (32) de la canule interne (3) par sa surface interne, de préférence à l'aide d'un adhésif de silicone, de préférence dans lequel la butée est constituée de telle sorte qu'une adaptation additionnelle d'éléments de connexion et/ou d'accessoires n'est pas entravée par la butée.

8. Agencement de tube trachéal ou de trachéostomie selon l'une des revendications 1 à 7, **caractérisé en ce qu'**un matériau de garniture opaque aux rayons X est pourvu dans la couche en matériau synthétique (21) et/ou dans la tresse (20), et/ou **en ce qu'**au moins la surface interne (31) de la canule interne (3) comporte une couche à effet antimicrobien, en particulier un revêtement à l'argent.

9. Procédé de fabrication d'une canule interne pour un agencement de tube trachéal ou de trachéostomie selon au moins l'une des revendications 1 et 2,
**caractérisé**
**en ce qu'**une structure de support tubulaire constituée d'une tresse (20) de filaments (35, 36) et/ou de fibres est pourvue, et
**en ce que** la couche en matériau synthétique (21) est réalisée en plongeant la tresse (20) dans un matériau synthétique liquide, de préférence un caoutchouc de silicone liquide, et en durcissant la couche en matériau synthétique (21).
